# EUROPÄISCHE PATENTSCHRIFT

(11) **EP 3 010 880 B1**
(45) Veröffentlichungstag und Bekanntmachung des Hinweises auf die Patenterteilung: **01.03.2017**
(21) Anmeldenummer: 14729332.8
(22) Anmeldetag: 11.06.2014
(51) Int. Cl.: C07C 209/26

(54) **VERFAHREN ZUR HERSTELLUNG VON 2-CHLORDIALKYLBENZYLAMINEN DURCH HYDRIERUNG**
METHOD FOR PRODUCING 2-CHLORODIALKYLBENZYLAMINES BY HYDROGENATION
PROCÉDÉ DE PRÉPARATION DE 2-CHLORODIALKYLBENZYLAMINES PAR HYDROGÉNATION

(30) Priorität: 21.06.2013 EP 13173233; 20.01.2014 EP 14151747
(43) Veröffentlichungstag der Anmeldung: 27.04.2016
(73) Patentinhaber: BASF SE, 67056 Ludwigshafen am Rhein (DE)
(72) Erfinder: WIGBERS, Christof Wilhelm, 68167 Mannheim (DE); ALTENHOFF, Ansgar Gereon, 69117 Heidelberg (DE); SIEMER, Michael, 68159 Mannheim (DE); MELDER, Johann-Peter, 67459 Böhl-Iggelheim (DE); EIDAMSHAUS, Christian, 68161 Mannheim (DE)
(74) Vertreter: BASF IP Association
(86) Internationale Anmeldenummer: PCT/EP2014/062068
(87) Internationale Veröffentlichungsnummer: WO 2014/202436

(56) Entgegenhaltungen:
- WO-A1-2013/017611
- BHATTACHARYYA SUKANTA: "A high throughput synthesis of N,N-dimethyl tertiary amines", SYNTHETIC COMMUNICATIONS: AN INTERNATIONAL JOURNAL FOR RAPID COMMUNICATION OF SYNTHETIC ORGANIC CHEMISTRY, TAYLOR & FRANCIS INC, PHILADELPHIA, PA; US, Bd. 30, Nr. 11, 1. Januar 2000 (2000-01-01), Seiten 2001-2008, XP002698158, ISSN: 0039-7911
- CARL D PERCHONOCK ET AL: "Facile synthesis of halo-substituted tetrahydroisoquinolines and tetrahydro-2-benzazepines via N-acetyl-1,2-dihydroisoquinolines", THE JOURNAL OF ORGANIC CHEMISTRY, AMERICAN CHEMICAL SOCIETY, US, Bd. 45, Nr. 10, 1. Mai 1980 (1980-05-01), Seiten 1950-1953, XP008166183, ISSN: 0022-3263, DOI: 10.1021/JO01298A038

## Beschreibung

Die Erfindung betrifft ein Verfahren zur Herstellung von 2-Chlordialkylbenzylaminen der Formel I 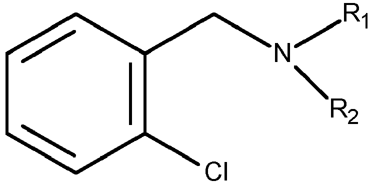 wobei R1 und R2 jeweils unabhängig voneinander für eine C1- bis C10 Alkylgruppe stehen, welches dadurch gekennzeichnet ist, dass 2-Chlorbenzaldehyd der Formel II 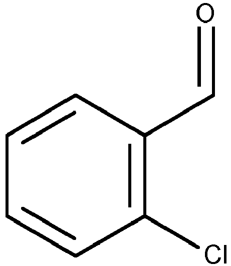 mit einem Dialkylamin der Formel III 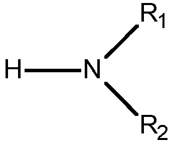 wobei R1 und R2 die obige Bedeutung haben,
und Wasserstoff umgesetzt werden, wobei die Umsetzung mit Wasserstoff in Gegenwart eines heterogenen Hydrierkatalysators, der Cobalt, Nickel, Kupfer oder Mischungen dieser drei Metalle in Mengen von insgesamt mindestens 5 Gew. % bezogen auf die Gewichtssumme aller aktiven Metalle des Hydrierkatalysators, enthält 2-Chlordimethylbenzylamin ist als Ausgangstoff für die Synthese von pharmazeutischen Wirkstoffen oder Pflanzenschutzmitteln von Bedeutung.

Aus WO 2013/017611 ist bekannt, 2-Chlordimethylbenzylamin durch Umsetzung von 2Chlorbenzylchlorid mit Dimethylamin herzustellen. Dabei werden neben 2-Chlordimethylbenzylamin chlor-haltige Salze erhalten. Für weitere Synthesen ist häufig eine vollständige Abtrennung der chlor-haltige Salze erforderlich. Nachteilig ist in diesem Zusammenhang insbesondere die gute Wasserlöslichkeit der chlor-haltigen Salze.

Auch DE 676331 beschreibt die Umsetzung von 2-Chlorbenzylchlorid und Dimethylamin zu 2-Chlordimethylbenzylamin; das anfallende Dimethylamin-Hydrochlorid wird als Schmelze abgetrennt. Auf diese Weise geht Dimethylamin verloren und kann nicht direkt erneut für die Umsetzung verwendet werden.

Die reduktive Carbonylaminierung von z. B. Acetophenon oder Formaldehyd mit Ameisensäure und einer Aminoverbindung ist als Leuckart-Wallach Reaktion bekannt geworden und in Lehrbüchern der organischen Chemie beschrieben (siehe Hans Beyer, Lehrbuch der organischen Chemie, s. Hirzel Verlag Stuttgart, 1978, Seite 454). S. H. Pine et al. beschreiben in J. Org. Chem., 1971, 36, 984-991 eine Leuckart-Wallach Reaktion von 2-Chlorbenylamin mit Ameisensäure und Formaldehyd.

S. Bhattacharyya (Synth. Comm., 2000, 30, 2001-2008) offenbart die reduktive Aminierung von Carbonylverbindungen mit Dimethylamine und die Reduktion des Kondensationsprodukts mit Natriumborhydrid.

Aufgabe der vorliegenden Erfindung war ein möglichst einfaches und wirtschaftliches Verfahren zur Herstellung von 2-Chlordimethylbenzylamin. Chlor-haltige Salze sollen bei dem Verfahren als Nebenprodukt möglichst vermieden werden. Gewünscht ist weiterhin ein möglichst hoher Umsatz zu 2-Chlordimethylbenzylamin.

Demgemäß wurde das eingangs beschriebene Verfahren gefunden.

Durch das erfindungsgemäße Verfahren werden 2-Chlordialkylbenzylamine der Formel I 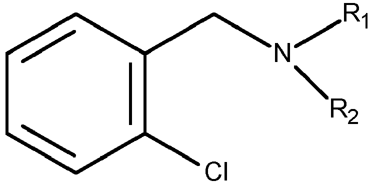 hergestellt.

Vorzugsweise stehen R1 und R2 unabhängig voneinander für eine C1- bis C4-Alkylgruppe. Besonders bevorzugt stehen R1 und R2 jeweils für eine Methylgruppe; bei der Verbindung der Formel I handelt es sich daher besonders bevorzugt um 2-Chlordimethylbenzylamin.

Zur Herstellung der 2-Chlordialkylbenzylamine wird 2-Chlorbenzaldehyd mit einem Dialkylamin der Formel III und mit Wasserstoff umgesetzt. R1 und R2 in Formel III entsprechen den Resten R1 und R2, bzw. den bevorzugten Resten R1 und R2 in Formel I.

Bei der Umsetzung reagieren 2-Chlorbenzaldehyd und Dialkylamin voraussichtlich zu einem Zwischenprodukt, wobei es z. B. um das entsprechende Aminal, Halbaminal oder Iminium-Ion handeln kann. Dieses Zwischenprodukt kann dann in Gegenwart von Wasserstoff zum 2-Chlordialkylbenzylamin hydriert werden.

Die Umsetzung kann in einer Verfahrensstufe bei gleichzeitiger Anwesenheit aller Reaktionspartner oder in zwei Verfahrensstufen erfolgen.

### Zur zweistufigen Ausführung:

In einem ersten Schritt kann 2-Chlorbenzaldehyd und das Dialkylamin umgesetzt und anschließend das Reaktionsprodukt in einem zweiten Verfahrensschritt mit Wasserstoff hydriert werden.

Die Umsetzung kann ohne Mitverwendung eines Lösemittels oder unter Verwendung eines Lösemittels durchgeführt werden; in einer bevorzugten Ausführungsform wird sie ohne Mitverwendung von Lösemittel durchgeführt.

Die Umsetzung kann z. B. in Gegenwart eines Lösemittels für das 2-Chlorbenzaldehyd durchgeführt werden Als Lösemittel für 2-Chlorbenzaldehyd geeignet sind insbesondere hydrophile, organische Lösemittel. Besonders bevorzugt sind hydrophile, nicht protische, organische Lösemittel. Genannt seien z.B. Ether, wie Tetrahydrofuran, Diethylether, Methyltertiärbutylether oder 1,4-Dioxan.

2-Chlorbenzaldehyd und das Lösemittel können dem Reaktionsgefäß als Lösung oder getrennt zugeführt werden.

In einer besonders bevorzugten Ausführungsform wird 2-Chlorbezaldehyd ohne Lösungsmittel eingesetzt.

Das Dialkylamin kann als Flüssigkeit, z. B. auch als Lösung in einem Lösemittel oder als Gas zugeführt werden. Dimethylamin hat z. B. einen Siedepunkt von 7°C und kann leicht als Gas zugeführt werden.

Alternativ kann es als z. B. Lösung in Wasser oder einem hydrophilen organischen Lösemittel, in dem das Dialkylamin mindestens teilweise löslich ist, zugeführt werden. Als Lösemittel kommen insbesondere Wasser, Ether, wie Tetrahydrofuran oder Alkohole wie Methanol oder Ethanol in Betracht.

Vorzugsweise wird das Dialkylamin ohne Verwendung Mitverwendung eines Lösemittels zugeführt. Besonders bevorzugt wird Dialkylamin gasförmig zugeführt.

Vorzugsweise wird das das Dialkylamin in mindestens equimolaren Mengen eingesetzt, insbesondere wird es in molarem Überschuss bezogen auf das 2-Chlorbenzaldehyd eingesetzt.

Insbesondere beträgt das Molverhältnis von Dialkylamin zum 2-Chlorbenzaldehyd 1 : 1 bis 50 : 1, besonders bevorzugt 2 : 1 bis 20 : 1 und ganz besonders bevorzugt 3 : 1 bis 15 : 1.

2-Chlorbenzaldehyd und Dialkylamin können zunächst in einem ersten Schritt ohne Gegenwart von Wasserstoff umgesetzt werden.

Die Umsetzung des 2-Chlorbenzaldehyds mit dem Dialkylamin kann z. B. bei Temperaturen von 20 bis 200°C, vorzugsweise 40 bis 120°C, besonders bevorzugt 60 bis 100°C erfolgen. Die Umsetzung kann z. B. bei Normaldruck oder Überdruck durchgeführt werden. In einer bevorzugten Ausführungsform wird sie bei erhöhtem Druck z. B. von 1,1 bis 200 bar, insbesondere 5 bis 100 bar und ganz besonders bevorzugt 40 bis 100 bar durchgeführt. Der Druck kann z. B. durch Aufpressen eines Gases, z. B. Wasserstoff oder ein Inertgases, insbesondere Stickstoff, eingestellt werden. Ohne Gegenwart eines Hydrierkatalysators erfolgt in dieser Stufe bei Verwendung von Wasserstoff zum Einstellen des Drucks noch keine Hydrierung.

Bei der anschließenden Hydrierung (zweite Stufe) wird der Wasserstoff gasförmig in ausreichenden Mengen, im Allgemeinen im molaren Überschuss bezogen auf 2-Chlorbenzaldehyd, zugeführt. Durch Aufrechthalten eines konstanten Wasserstoffdrucks wird verbrauchter Wasserstoff nachgeführt, so dass Wasserstoff immer in ausreichenden Mengen für die Hydrierung zur Verfügung steht.

Die Umsetzung mit Wasserstoff erfolgt in Gegenwart eines Katalysators für die Hydrierung.

Der Katalysator liegt als Feststoff vor, es handelt sich daher um einen heterogenen Katalysator.

Heterogene Katalysatoren für die Hydrierung sind katalytisch aktive Elemente oder Verbindungen, die als Feststoff in beliebiger Form vorliegen; sie können z. B. selbst in Teilchenform vorliegen, sie können auf einen Träger, z. B. aus Calciumcarbonat, Siliciumoxid, Zirkoniumdioxid oder Aluminiumdioxid, oder auf Oberflächen im Reaktor aufgebracht sein.

Die Hydrierkatalysatoren enthalten aktive Metalle, entweder in elementarer Form oder in Form von Verbindungen, z. B. Oxiden. Oft enthalten die Katalysatoren Mischungen von aktiven Metallen. Der Begriff Metall umfasst daher im Folgenden elementare Metalle oder auch Metalle, wie sie in chemischen Bindungen, sei es in ionischer Form oder kovalent gebundener Form vorliegen. Bei Verwendung der aktiven Metalle in Form ihrer Oxide oder gegebenenfalls auch sonstiger Verbindungen erfolgt im Allgemeinen bei höheren Temperaturen, insbesondere in Gegenwart von Wasserstoff, eine Reduktion der Oxide zu den Metallen. Dies kann mit Beginn der Umsetzung geschehen oder in einem separaten Schritt vorher durchgeführt werden.

Die Hydrierkatalysatoren enthalten Cobalt, Nickel, Kupfer oder Mischungen dieser drei Metalle in Mengen von insgesamt mindestens 5 Gew. %, besonders bevorzugt mindestens 20 Gew. % und ganz besonders bevorzugt mindestens 50 Gew. %, bezogen auf die Gewichtssumme aller aktiven Metalle des Hydrierkatalysators.

Die vorstehenden Metalle können elementar oder als Verbindung, z. B. als Oxide vorliegen. Als Katalysatoren, welche Metalle in ihrer elementaren Form enthalten seien Raney-Nickel oder Raney-Cobalt erwähnt.

Der Katalysator kann in dem Reaktor z. B. als Festbett vorliegen.

Die Hydrierung erfolgt vorzugsweise bei einer Temperatur von 20 bis 200°C, besonders bevorzugt von 40 bis 120°C, ganz besonders bevorzugt 60 bis 100°C. Die Hydrierung wird vorzugsweise bei einem erhöhten Druck z. B. von 1,1 bis 200 bar, insbesondere 5 bis 100 bar und ganz besonders bevorzugt 40 bis 100 bar durchgeführt. Vorzugsweise handelt es sich dabei um den eingestellten Wasserstoffdruck oder den Druck eines Gasgemisches aus Wasserstoff und einem Inertgas.

Bei Verwendung von Wasser oder eines hydrophilen Lösemittel für das Dialkylamin kann das Reaktionsprodukt zweiphasig sein. Die organische Phase enthält das erhaltene 2- Chlordimethylbenzylamin, nicht umgesetztes 2-Chlorbenzaldehyd und gegebenenfalls darin lösliche Nebenprodukte.

Die wässrige, bzw. hydrophile Phase enthält das Lösemittel, in dem das Dialkylamin gelöst war (Wasser bzw. ein hydrophiles Lösemittel), nicht umgesetztes Dialkylamin und gegebenenfalls darin lösliche Nebenprodukte.

Die organische Phase kann leicht abgetrennt werden. Die Reinigung oder Abtrennung der nicht umgesetzten Ausgangsstoffe (2-Chlorbenzaldehyd) kann z. B. durch Destillation oder Rektifikation erfolgen.

Ohne Verwendung von Wasser oder eines hydrophilen Lösemittel für das Dialkylamin wird im Allgemeinen nur eine organische Phase erhalten, welche 2-Chlordialkylbenzylamin, nicht umgesetztes 2-Chlorbenzaldehyd und gegebenenfalls darin lösliche Nebenprodukte enthält. Das Dia-Ikylamin kann durch Destillation oder Rektifikation abgetrennt und, wenn gewünscht, in die Umsetzung zurückgeführt werden. Dimethylamin (Siedepunkt 7°C), ist bei entsprechenden Temperaturen flüchtig und kann besonders leicht bereits abgetrennt werden (Entgasung). Die organische Phase kann gegebenenfalls durch Destillation oder Rektifikation weiter aufgearbeitet bzw. gereinigt werden.

### Zur einstufigen Ausführung

In einer weiteren bevorzugten Ausführungsform kann die Umsetzung einstufig durchgeführt werden. Dazu wird 2-Chlorbenzaldehyd mit dem Dialkylamin in Gegenwart von Wasserstoff und vorzugsweise in Gegenwart eines Katalysators für die Hydrierung umgesetzt. Der Katalysator für die Hydrierung kann in dem Reaktor z. B. als Festbett eingebracht sein.

Für die einstufige Ausführungsform gelten alle vorstehenden Angaben entsprechend. Insbesondere kann die einstufige Ausführungsform, wie oben ausgeführt in Abwesenheit oder Anwesenheit von Lösemittel durchgeführt werden.

Die einstufige Umsetzung erfolgt vorzugsweise bei einer Temperatur von 20 bis 200°C, besonders bevorzugt von 40 bis 120°C, ganz besonders bevorzugt 60 bis 100°C sowie bei einem erhöhten Druck z. B. von 1,1 bis 200 bar, insbesondere 5 bis 100 bar und ganz besonders bevorzugt 40 bis 100 bar durchgeführt. Vorzugsweise handelt es sich dabei um den eingestellten Wasserstoffdruck oder den Druck eines Gasgemisches aus Wasserstoff und einem Inertgas.

Sowohl die einstufige als auch die zweistufige Umsetzung kann diskontinuierlich (batch-Verfahren), halbkontinuierlich oder kontinuierlich erfolgen.

Bei der halbkontinuierlichen Ausführungsform wird mindestens ein Ausgangsstoff vollständig vorgelegt und mindestens ein Ausgangastoff während der Umsetzung über einen längeren Zeitraum kontinuierlich zugefahren.

Bei der kontinuierlichen Ausführungsform werden die Ausgangsstoffe kontinuierlich zugeführt und die Produkte kontinuierlich abgeführt. Die kontinuierliche Ausführungsform eignet sich sowohl für die oben beschriebene einstufige als die oben beschriebene zweistufige Durchführung.

In einer bevorzugten Ausführungsform wird Umsetzung kontinuierlich durchgeführt. Die kontinuierliche Umsetzung kann z.B. in einem Rührkessel, einer Rührkesselkaskade oder einem Rohreaktor durchgeführt werden.

In einer besonders bevorzugten Ausführungsform wird die Umsetzung kontinuierlich in einer Stufe durch Umsetzung von 2-Chlorbenzaldehyd mit Dialkylamin in Gegenwart von Wasserstoff durchgeführt. Dazu werden 2-Chlorbenzaldehyd und das Dialkylamin oder deren Lösungen einem Reaktor kontinuierlich zugeführt. Der Reaktor enthält den Hydrierkatalysator. Der Wasserstoffdruck bzw. Wasserstoffpartialdruck wird während der Umsetzung aufrecht gehalten. Auch die anschließende Aufarbeitung kann kontinuierlich erfolgen.

Durch das erfindungsgemäße Verfahren sind 2-Chlordialkylbenzylamine in einfacher und wirtschaftlicher Weise erhältlich. Chlor-haltige Salze fallen nicht als Nebenprodukt an. Das Verfahren ermöglicht die Herstellung von 2-Chlordialkylbenzylaminen, insbesondere 2-Chlordimethylbenzylaminen in hoher Ausbeute und Selektivität.

### Beispiele

In den nachstehenden Beispielen wurden Hydrierkatalysatoren verwendet, welche die aktiven Metalle in ihrer oxidischen Form enthielten. In den unten stehenden Beispielen 1 bis 4 sind die aktiven Metalle angegeben. Die Umwandlung der jeweiligen Metalloxide in die Metallform erfolgt bei Beginn der Umsetzung (Aktivierung).

### Beispiel 1 (THF - Co-haltiger Katalysator, 80 °C, 80 bar)

Ein 0.3 Liter Rührautoklav wurde mit einem heterogenen Co/Mn₄/Na-haltigen Katalysator (20 g) bestückt und mit 2-Chlorbenzaldehyd (19.0 g, 0.14 mol) und THF (79 mL) befüllt. Dimethylamin (45.0 g, 1.0 mol) wurde aufgepresst und zum Eigendruck wurden 10 bar N₂ gepresst. Die Reaktionsmischung wurde sechs Stunden bei 80 °C gerührt bevor Wasserstoff auf 80 bar aufgepresst wurde. Die Reaktion wurde bei 80 °C gerührt und der Druck durch Zugabe von Wasserstoff bei 80 bar gehalten. Nach sechs Stunden wurde auf Raumtemperatur abgekühlt und auf Normaldruck entspannt. Der Reaktionsaustrag wurde am Rotationsverdampfer eingeengt und der Rückstand durch Gaschromatographie analysiert. Die Ausbeute (gemessen in GC-Gew.-%) an 2-Chlordimethylbenzylamin betrug 90%.

### Beispiel 2 (THF - Co/Ni/Cu/Sn-haltiger Katalysator (a), 80 °C, 80 bar)

Ein 0.3 Liter Rührautoklav wurde mit einem heterogenen Co/Ni/Cu/Sn-haltigen Katalysator geträgert auf Al₂O₃ (20 g) bestückt und mit 2-Chlorbenzaldehyd (19.0 g, 0.14 mol) und THF (79 mL) befüllt. Dimethylamin (45.0 g, 1.0 mol) wurde aufgepresst und zum Eigendruck wurden 10 bar N₂ gepresst. Die Reaktionsmischung wurde sechs Stunden bei 80 °C gerührt bevor Wasserstoff auf 80 bar aufgepresst wurde. Die Reaktion wurde bei 80 °C gerührt und der Druck durch Zugabe von Wasserstoff bei 80 bar gehalten. Nach sechs Stunden wurde auf Raumtemperatur abgekühlt und auf Normaldruck entspannt. Der Reaktionsaustrag wurde am Rotationsverdampfer eingeengt und der Rückstand durch Gaschromatographie analysiert. Die Ausbeute (gemessen in GC-Gew.-%) an 2-Chlordimethylbenzylamin betrug 71%.

### Beispiel 3 (THF - Co/Ni/Cu-haltiger Katalysator (b), 80 °C, 80 bar)

Ein 0.3 Liter Rührautoklav wurde mit einem heterogenen Co/Ni/Cu-haltigen Katalysator geträgert auf ZrO₂ (20 g) bestückt und mit 2-Chlorbenzaldehyd (19.0 g, 0.14 mol) und THF (79 mL) befüllt. Dimethylamin (45.0 g, 1.0 mol) wurde aufgepresst und zum Eigendruck wurden 10 bar N₂ gepresst. Die Reaktionsmischung wurde sechs Stunden bei 80 °C gerührt bevor Wasserstoff auf 80 bar aufgepresst wurde. Die Reaktion wurde bei 80°C gerührt und der Druck durch Zugabe von Wasserstoff bei 80 bar gehalten. Nach sechs Stunden wurde auf Raumtemperatur abgekühlt und auf Normaldruck entspannt. Der Reaktionsaustrag wurde am Rotationsverdampfer eingeengt und der Rückstand durch Gaschromatographie analysiert. Die Ausbeute (gemessen in GC-Gew.-%) an 2-Chlordimethylbenzylamin betrug 96%.

### Beispiel 4 (lösungsmittelfreie Reaktionsführung - Co/Ni/Cu-haltiger Katalysator (b), 60°C, 40 bar)

Ein 0.3 Liter Rührautoklav wurde mit einem heterogenen Co/Ni/Cu-haltigen Katalysator geträgert auf ZrO₂ (20 g) bestückt und mit 2-Chlorbenzaldehyd (19.0 g, 0.14 mol) befüllt. Dimethylamin (45.0 g, 1.0 mol) wurde aufgepresst und zum Eigendruck wurden 10 bar N₂ gepresst. Die Reaktionsmischung wurde sechs Stunden bei 80 °C gerührt. Die Autoklaven-Temperatur wurde auf 60 °C verringert und Wasserstoff auf 40 bar aufgepresst. Die Reaktion wurde bei 60 °C gerührt und der Druck durch Zugabe von Wasserstoff bei 40 bar gehalten. Nach sechs Stunden wurde auf Raumtemperatur abgekühlt und auf Normaldruck entspannt. Der Reaktionsaustrag wurde am Rotationsverdampfer eingeengt und der Rückstand durch Gaschromatographie analysiert. Die Ausbeute (gemessen in GC-Gew.-%) an 2-Chlordimethylbenzylamin betrug 91 %.

### Vergleichsbeispiel 5 (lösungsmittelfreie Reaktionsführung - Pd-haltiger Katalysator, 60 °C, 40 bar)

Ein 0.3 Liter Rührautoklav wurde mit einem heterogenen Pd/Ag-haltigen Katalysator geträgert auf Al₂O₃ (10 g) bestückt und mit 2-Chlorbenzaldehyd (90.0 g, 0.64 mol) befüllt. 10 bar Wasserstoff wurden aufgepresst gefolgt von Dimethylamin (45.0 g, 0.98 mol). Die Reaktionsmischung wurde auf 100 °C erwärmt und Wasserstoff auf 90 bar aufgepresst. Die Reaktion wurde bei 100 °C gerührt und der Druck durch Zugabe von Wasserstoff bei 90 bar gehalten. Nach sechs Stunden wurde auf Raumtemperatur abgekühlt und auf Normaldruck entspannt. Die Reaktionsmischung wurde durch Gaschromatographie analysiert. Die Ausbeute (gemessen in GC-Flächen-%) an 2-Chlordimethylbenzylamin betrug 41 %.

### GC-Bedingungen:

1) Für Beispiele 1-4: 30 m RTX 200 Säule, 60 °C/5 min. → 280 °C - 8 °C/min., 30 min.
2) Für Beispiel 5: 30 m DB5 Säule, 80 °C/3 min. → 280 °C - 10 °C/min., 30 min.

### Beispiel 6 (THF- Cu-Katalysator)

Ein 0,3 Liter Rührautoklav wurde mit einem heterogenen CuO-haltigen Katalysator geträgert auf Al2O3 (20 g) bestückt und mit 2-Chlorbenzaldehyd (19,0 g, 0,14 mol) und THF (79 mL) befüllt.

Dimethylamin (45,0 g, 1.0 mol) wurde aufgepresst und zum Eigendruck wurden 10 bar N2 gepresst. Die Reaktionsmischung wurde sechs Stunden bei 80 °C gerührt. Die Autoklaven-Temperatur wurde auf 60 °C verringert und Wasserstoff auf 40 bar aufgepresst. Die Reaktion wurde bei 60 °C gerührt und der Druck durch Zugabe von Wasserstoff bei 40 bar gehalten. Nach sechs Stunden wurde auf Raumtemperatur abgekühlt und auf Normaldruck entspannt. Der Reaktionsaustrag wurde am Rotationsverdampfer eingeengt und der Rückstand durch Gaschromatographie analysiert. Die Ausbeute (gemessen in GC-Gew.-%) an 2-Chlordimethylbenzylamin betrug 99,2%.

### Beispiel 7 (THF- Cu/La haltiger Katalysator)

Ein 0,3 Liter Rührautoklav wurde mit einem heterogenen CuO- und La2O3-haltigen Katalysator geträgert auf Al2O3 (20 g) bestückt und mit 2-Chlorbenzaldehyd (19,0 g, 0,14 mol) und THF (79 mL) befüllt. Dimethylamin (45,0 g, 1,0 mol) wurde aufgepresst und zum Eigendruck wurden 10 bar N2 gepresst. Die Reaktionsmischung wurde sechs Stunden bei 80 °C gerührt. Die Autoklaven-Temperatur wurde auf 60 °C verringert und Wasserstoff auf 40 bar aufgepresst. Die Reaktion wurde bei 60 °C gerührt und der Druck durch Zugabe von Wasserstoff bei 40 bar gehalten. Nach sechs Stunden wurde auf Raumtemperatur abgekühlt und auf Normaldruck entspannt. Der Reaktionsaustrag wurde am Rotationsverdampfer eingeengt und der Rückstand durch Gaschromatographie analysiert. Die Ausbeute (gemessen in GC-Gew.-%) an 2-Chlordimethylbenzylamin betrug 99,0%.

## Patentansprüche

1. Verfahren zur Herstellung von 2-Chlordialkylbenzylaminen der Formel I 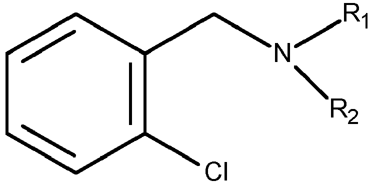 wobei R1 und R2 jeweils unabhängig voneinander für eine C1- bis C10 Alkylgruppe stehen,
**dadurch gekennzeichnet, dass** 2-Chlorbenzaldehyd der Formel II 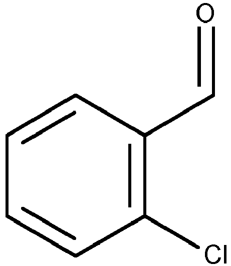 mit einem Dialkylamin der Formel III 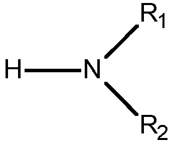 wobei R1 und R2 die obige Bedeutung haben,
und Wasserstoff umgesetzt wird, wobei die Umsetzung mit Wasserstoff in Gegenwart eines heterogenen Hydrierkatalysators, der Cobalt, Nickel, Kupfer oder Mischungen dieser drei Metalle in Mengen von insgesamt mindestens 5 Gew. % bezogen auf die Gewichtssumme aller aktiven Metalle des Hydrierkatalysators, enthält

2. Verfahren gemäß Anspruch 1, **dadurch gekennzeichnet, dass** R1 und R2 für eine Methylgruppe stehen

3. Verfahren gemäß Anspruch 1 oder 2, **dadurch gekennzeichnet, dass** das Molverhältnis von 2-Chlorbenzaldehyd zum Dialkylamin 1 : 1 bis 1: 20 beträgt

4. Verfahren gemäß einem der Ansprüche 1 bis 3, **dadurch gekennzeichnet, dass** zunächst 2-Chlorbenzaldehyd und das Dialkylamin umgesetzt werden und anschließend das Reaktionsprodukt mit Wasserstoff hydriert wird.

5. Verfahren gemäß einem der Ansprüche 1 bis 4, **dadurch gekennzeichnet, dass** die Umsetzung kontinuierlich durchgeführt wird.

6. Verfahren gemäß einem der Ansprüche 1 bis 5, **dadurch gekennzeichnet, dass** die Umsetzung kontinuierlich durch Umsetzung von 2-Chlorbenzaldehyd mit Dialkylamin in Gegenwart von Wasserstoff durchgeführt wird.

## Claims

1. Process for preparing 2-chlorodialkylbenzylamines of the formula I 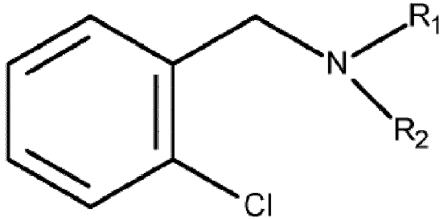 where R1 and R2 are each, independently of one another, a C1-C10-alkyl group,
**characterized in that** 2-chlorobenzaldehyde of the formula II 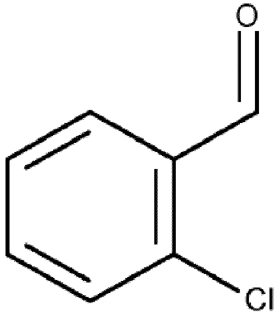 is reacted with a dialkylamine of the formula III 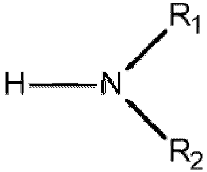 where R1 and R2 are as defined above,
and hydrogen, where the reaction with hydrogen is carried out in the presence of a heterogeneous hydrogenation catalyst comprising cobalt, nickel, copper or mixtures of these three metals in total amounts of at least 5% by weight based on the total weight of all active metals of the hydrogenation catalyst.

2. Process according to Claim 1, **characterized in that** R1 and R2 are each a methyl group.

3. Process according to Claim 1 or 2, **characterized in that** the molar ratio of 2-chlorobenzaldehyde to the dialkylamine is from 1:1 to 1:20.

4. Process according to any of Claims 1 to 3, **characterized in that** 2-chlorobenzaldehyde and the dialkylamine are reacted first and the reaction product is subsequently hydrogenated by means of hydrogen.

5. Process according to any of Claims 1 to 4, **characterized in that** the reaction is carried out continuously.

6. Process according to any of Claims 1 to 5, **characterized in that** the reaction is carried out continuously by reaction of 2-chlorobenzaldehyde with dialkylamine in the presence of hydrogen.

## Revendications

1. Procédé de fabrication de 2-chlorodialkylbenzylamines de formule I 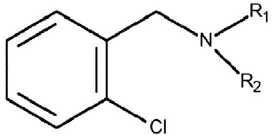 dans laquelle R1 et R2 représentent chacun indépendamment l'un de l'autre un groupe alkyle en C1 à C10,
**caractérisé en ce que** du 2-chlorobenzaldéhyde de formule II 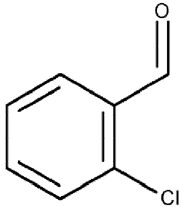 est mis en réaction avec une dialkylamine de formule III 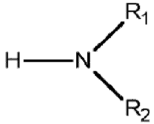 dans laquelle R1 et R2 ont la signification précédente, et de l'hydrogène, la réaction avec l'hydrogène ayant lieu en présence d'un catalyseur d'hydrogénation hétérogène qui contient du cobalt, du nickel, du cuivre ou des mélanges de ces trois métaux en quantités totales d'au moins 5 % en poids, par rapport à la somme en poids de tous les métaux actifs du catalyseur d'hydrogénation.

2. Procédé selon la revendication 1, **caractérisé en ce que** R1 et R2 représentent un groupe méthyle.

3. Procédé selon la revendication 1 ou 2, **caractérisé en ce que** le rapport molaire entre le 2-chlorobenzaldéhyde et la dialkylamine est de 1:1 à 1:20.

4. Procédé selon l'une quelconque des revendications 1 à 3, **caractérisé en ce que** le 2-chlorobenzaldéhyde et la dialkylamine sont tout d'abord mis en réaction, puis le produit de la réaction est hydrogéné avec de l'hydrogène.

5. Procédé selon l'une quelconque des revendications 1 à 4, **caractérisé en ce que** la réaction est réalisée en continu.

6. Procédé selon l'une quelconque des revendications 1 à 5, **caractérisé en ce que** la réaction est réalisée en continu par mise en réaction de 2-chlorobenzaldéhyde avec une dialkylamine en présence d'hydrogène.
